# EUROPÄISCHE PATENTSCHRIFT

(11) **EP 1 522 274 B1**
(45) Veröffentlichungstag und Bekanntmachung des Hinweises auf die Patenterteilung: **24.05.2017**
(21) Anmeldenummer: 04030672.2
(22) Anmeldetag: 17.02.1998
(51) Int. Cl.: A61F 2/06

(54) **Stent**
Stent
Stent

(30) Priorität: 17.02.1997 DE 29702671 U
(43) Veröffentlichungstag der Anmeldung: 13.04.2005
(62) Teilanmeldung aus: 98909471.9
(73) Patentinhaber: Abbott Laboratories Vascular Enterprises Limited, Terrace, Dublin 2 (IE)
(72) Erfinder: Von Oepen, Randolf, Los Altos Hilles CA 94024 (US)
(74) Vertreter: Hoefer & Partner Patentanwälte mbB

(56) Entgegenhaltungen:
- WO-A-96/03092
- WO-A-97/32543
- DE-U- 29 608 037
- DE-U- 29 615 969
- DE-U- 29 708 879
- DE-U- 29 716 476
- US-A- 5 697 971

## Beschreibung

Die Erfindung betrifft einen Stent gemäß Anspruch 1.

Unter einem Stent versteht man eine medizinisch-technische Vorrichtung, die an die Stelle von Verengungen von Körpergefäßen oder Körperhöhlungen eingeführt wird und dort im aufgeweiteten Zustand die Verengung aufweitet und im aufgeweiteten Zustand hält. Hierzu muß ein Stent im nicht-expandierten Zustand äußerst flexibel sein, damit er beim Einführen in die Körpergefäße deren Windungen problemlos folgen kann. Ferner muß der Stent im aufgeweiteten Zustand ausreichend stabil sein, um das gewünschte Aufweitungsmaß aufrechterhalten zu können.

Obwohl der Stent gemäß der DE 296 08 037.1 diese Anforderungen bereits zufriedenstellend erfüllt, ist grundsätzlich eine weitere Verbesserung der Stenteigenschaften wünschenswert.

Aus der WO 96/03092 A und der DE 296 15 969 U sind jeweils Stentkonstruktionen bekannt, bei denen der rohrförmige flexible Körper des jeweiligen Stents durch mäandrierende Stegmuster aufgebaut ist, die über Stegschleifen miteinander verbunden sind.

Es ist daher Aufgabe der vorliegenden Erfindung, einen Stent zu schaffen, dessen Eigenschaften hinsichtlich Flexibilität im nicht-expandierten Zustand und Formstabilität im expandierten Zustand weiter verbessert sind.

Die Lösung dieser Aufgabe erfolgt durch die Merkmale des Anspruches 1.

Der erfindungsgemäße Stent zeichnet sich durch eine Verbesserung der an sich gegenläufigen Eigenschaften hoher Flexibilität im nicht-expandierten Zustand und hoher Formstabilität im expandierten Zustand aus.

Mit dem erfindungsgemäßen Stent wird eine Konstruktion geschaffen, deren Stegstruktur eine Vielzahl von aneinander angrenzenden Zellen aufweist. Die Stegstruktur kann bei unterschiedlichen Ausführungsformen aus unterschiedlich aufgebauten Stegen bzw. Schenkeln bestehen, die Zellen umgrenzen. Erfindungsgemäß ist pro Zelle zumindestens ein Federelement vorgesehen. Das Federelement kann beispielsweise eine U- oder V-förmige Schleife in einem der Stege bzw. Schenkel jeder Zelle sein. Somit ist es mit der Erfindung möglich, einen Multizellularstent zu bilden, der eine Vielzahl von beispielsweise rautenförmigen oder zumindest rautenähnlichen Zellen aufweist. Die die Zellen bzw. Rauten umgrenzenden Schenkel bzw. Stege können hierbei die Federelemente aufweisen, welche im nichtgespreizten Zustand ausreichende Flexibilität über der Längsachse möglich machen.

Die Unteransprüche haben vorteilhafte Weiterbildungen der Erfindung zum Inhalt.

Weitere Einzelheiten, Merkmale und Vorteile der Erfindung ergeben sich aus nachfolgender Beschreibung von Ausführungsbeispielen anhand der Zeichnung. Es zeigt:
- Fig. 1: eine schematisch stark vereinfachte Darstellung des Grundaufbaus eines Stents,
- Fig. 2A: eine der Fig. 1 entsprechende Darstellung der Stegstruktur der Wand des Stents gemäß Fig. 1,
- Fig. 2B: eine der Fig. 2A entsprechende Darstellung einer Abwandlung der Stegstruktur der Wand des Stents gemäß Fig. 2A,
- Fig. 3: eine der Fig. 2 entsprechende Darstellung einer alternativen Ausführungsform einer Stegsstruktur,
- Fig. 4: die Stegstruktur gemäß Fig. 3 in aufgeweitetem Zustand,
- Fig. 5: eine Variante der Ausführungsform gemäß Fig. 2,
- Fig. 6: eine weitere Variante der Ausführungsform gemäß Fig. 2,
- Fig. 7: eine der Fig. 2 entsprechende Darstellung einer weiteren Ausführungsform einer Stegstruktur der Wand des Stents gemäß Fig. 1,
- Fig. 8: eine der Fig. 7 entsprechende Darstellung einer weiteren Ausführungsform,
- Fig. 9: eine der Fig. 8 entsprechende Darstellung einer Abwandlung der Ausführungsform der Stegstruktur gemäß Fig. 8, und
- Fig. 10: eine Abwandlung der Ausführungsform von Fig. 9.

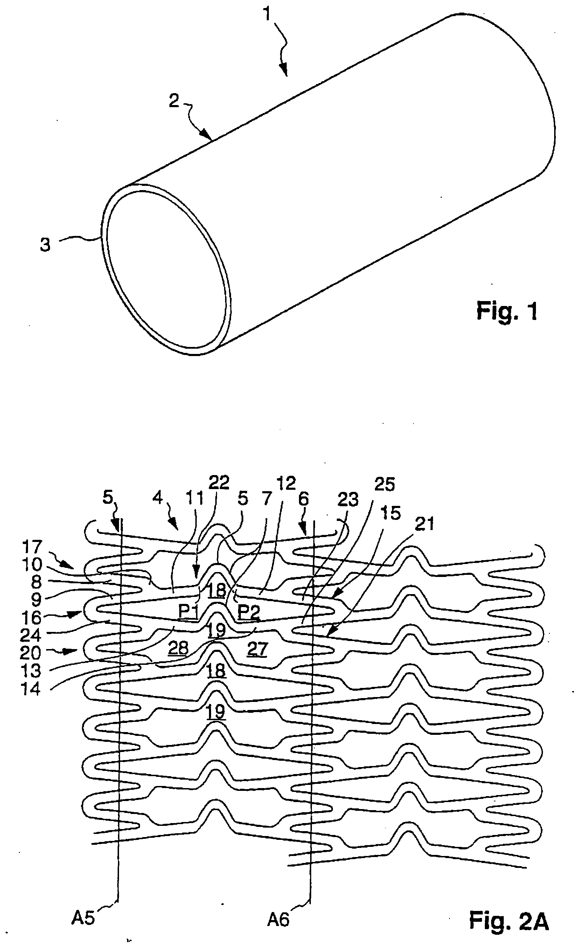

Fig. 1 zeigt den grundsätzlichen Aufbau eines Stents 1, der einen flexiblen, rohrförmigen Körper 2 mit einer Wand 3 aufweist, von der in Fig. 1 die Stirnansicht dargestellt ist.

Fig. 2A verdeutlicht den Aufbau einer Stegstruktur 4, die die Wand 3 bildet, wobei die Stegstruktur 4 jedoch in Fig. 1 zur Vereinfachung der zeichnerischen Darstellung nicht im einzelnen gezeigt ist. Daher wird nachfolgend betreffend den Aufbau der Stegstruktur 4 ausschließlich auf Fig. 2A Bezug genommen.

Die Stegstruktur 4 weist je nach Größe des Stents 1 eine Mehrzahl nebeneinander angeordneter gewundener (auch mäanderförmig genannter) Stegmuster auf, von denen in Fig. 2A repräsentativ die Stegmuster 5 und 6 mit Bezugszeichen gekennzeichnet sind. Wie in Fig. 1 verdeutlicht, verlaufen die Stegmuster 5, 6 gewunden und sind derart ausgerichtet, daß ihre auf den Zylindermantelflächen des Körpers 2 senkrecht stehende Achsen A₅ und A₆ parallel zueinander angeordnet sind.

Die Stegmuster (von denen nachfolgend repräsentativ immer nur auf die Stegmuster 5, 6 Bezug genommen wird) weisen jeweils eine Mehrzahl von Stegschleifen auf, von denen in Fig. 2A die Stegschleifen 15, 16 und 17 mit Bezugsziffern versehen sind. Wie sich aus dem gewundenen Verlauf ergibt, öffnen sich gemäß der in Fig. 2A gewählten Darstellung diese Stegschleifen alternatierend nach links und rechts.

Die Stegmuster 5, 6 spannen jeweils über Federelemente Zellen auf, wobei ein Federelement repräsentativ in Fig. 2A dargestellt und in seiner Gesamtheit mit der Bezugsziffer 22 versehen ist. Das Element 22 weist zwei geradlinig verlaufende Stege 11, 12 und einen haarnadelartig ausgebildeten Stegbogen 7 auf. Der Stegbogen 7 weist einen Scheitelpunkt S und Fußpunkte P₁, P₂ auf. Die geradlinig verlaufenden Stege 11, 12 schließen sich an die Fußpunkte P₁ bzw. P₂ an und verlaufen zu den jeweils benachbarten Stegmustern, im in Fig. 2A dargestellten Zustand also zum Stegmuster 5 (Steg 11) bzw. zum Stegmuster 6 (Steg 12).

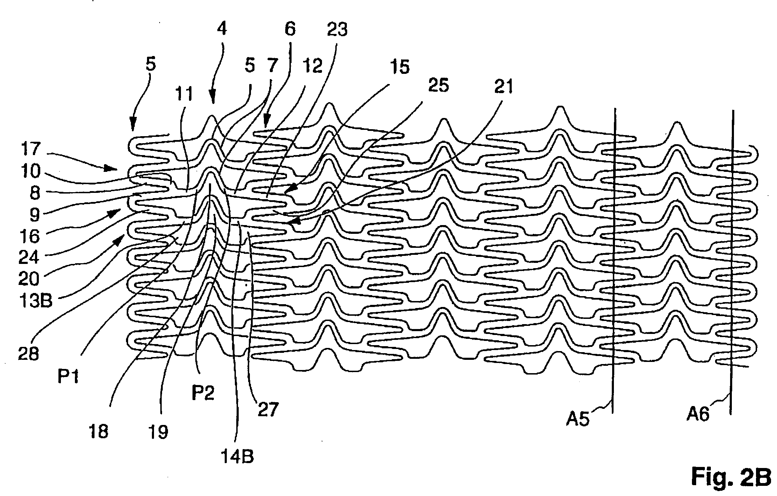

Wie Fig. 2A ferner verdeutlicht, weist jede Stegschleife wiederum jeweils zwei geradlinige Stege 8, 9 und einen diese verbindenden gebogenen Stegabschnitt 10 auf. Die Stege 8 und 9 schließen hierbei einen spitzen Winkel ein. Im Falle der Stege 11 und 12 gehen diese geradlinig in den Steg 9 der Stegschleife 17 (Steg 11) bzw. den Steg 23 der Stegschleife 15 (Steg 12) des Stegmusters 6 über. Im Falle des in Fig. 1 sich nach unten nächsten anschließenden Elementes geht dessen linker Steg 13 in den Steg 24 der Stegschleife 16 über. Der auf der anderen Seite des nächsten Stegbogens 7 angeordnete Steg 14 geht in den Steg 25 der Stegschleife 15 über. Wie Fig. 2 verdeutlicht, weisen hierbei die Stege 11 und 12 leicht nach unten, während die Stege 13 und 14 leicht nach oben weisen.

Die Stege 11, 12, 13 und 14 sowie die beiden zugeordneten Stegbögen 7 umschließen hierbei eine Zelle bzw. eine Kammer 18. Die darunterliegende Kammer 19 wird wiederum von Stegen und Stegbögen begrenzt. Aufgrund des Umstandes, daß die zwei Stegschleifen 20 und 21 im Übergangsbereich zwischen den Stegen der Elemente und den Stegen der zugeordneten Stegschleifen abgehen, ergibt sich eine andere Form einer Kammer 19, die zu beiden Seiten des Stegbogens 7 erweiterte Kammerbereiche 27 und 28 aufweist. Fig. 2A verdeutlicht hierbei, daß die Kammern 18 und 19 jeweils abwechselnd aufeinander folgen, wobei die Kammerbereiche 27 und 28 jeweils im Vergleich zu den Kammerbereichen der Kammer 18 rechts und links vom zugeordneten Stegbogen 7 vergrößert sind.

Fig. 2A verdeutlicht ferner, daß die Scheitelpunkte S aller Stegbögen 7 (in Fig. 2 ist lediglich ein Stegbogen 7 mit einem zugeordneten Scheitelpunkt S repräsentativ mit Bezugsziffern versehen) alle in die gleiche Richtung weisen. Es ist hierbei möglich, daß die Stegbögen 7 alle gleich groß oder auch vorzugsweise alternierend unterschiedlich groß bzw. hoch und weit sind.

In Fig. 2B ist eine Abwandlung der Ausführungsform gemäß Fig. 2A dargestellt. Im Prinzip entspricht die Ausführungsform gemäß Fig. 2B der Fig. 2A, so daß alle übereinstimmenden Teile mit den gleichen Bezugsziffern wie in Fig. 2A versehen sind. Insofern kann auf die vorangehende Beschreibung zu Fig. 2A verwiesen werden. Der Unterschied zwischen den Ausführungsformen gemäß Fig. 2A und Fig. 2B besteht darin, daß alle Stege zu den Stegbögen 7 gemäß der gewählten Darstellung in Fig. 2B nach oben verlaufen. Zur Verdeutlichung des Unterschiedes zwischen den Ausführungsformen gemäß Fig. 2A und Fig. 2B ist in Fig. 2B das Stegpaar 13 mit dem Index "B" versehen. Mit anderen Worten bedeutet dies, daß die Stege zu den Stegbögen 7 alle in die gleiche Richtung weisen, beispielsweise alle nach oben, so daß sie alle parallel zueinander liegen. In der Ausführungsform gemäß Fig. 2A verlaufen die Stege hingegen abwechselnd nach oben und nach unten. Die Ausführungsform gemäß Fig. 2B ergibt den Vorteil, daß die von den Stegen aufgespannten Zellen 18, 19 über den Umfang und über die Länge jeweils gleich sind. Ergänzend zur schriftlichen Offenbarung wird zur Offenbarung des Stegmusters bzw. der Stegstruktur der Fig. 2B explizit auf die zeichnerische Darstellung verwiesen.

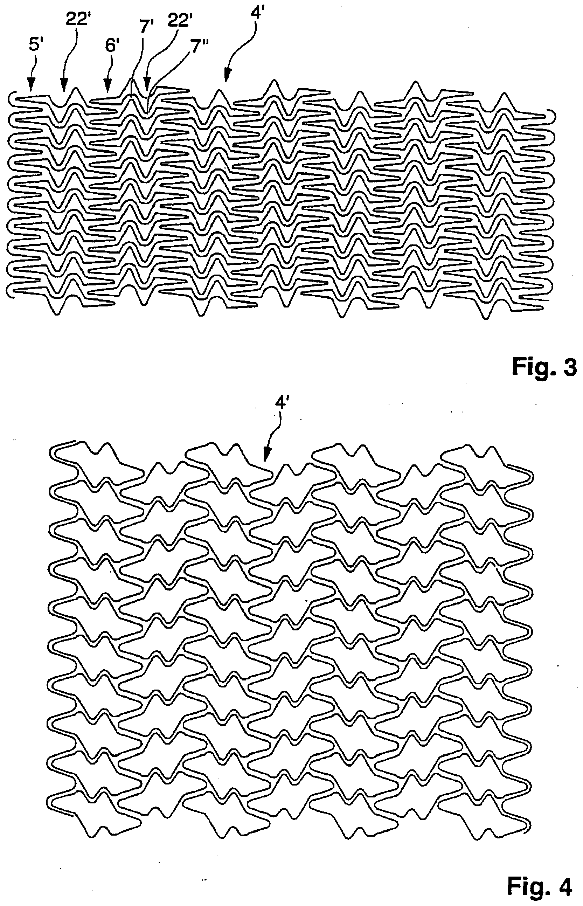

In Fig. 3 ist eine alternative Ausführungsform einer Stegstruktur 4' dargestellt, die wiederum nebeneinander mäanderartig verlaufende Stegmuster 5' und 6' aufweist, die mit Federelementen 22' eine Zelle aufspannen. Die Elemente 22' weisen bei dieser Ausführungsform S-förmige Federabschnitte und somit sich aneinander anreihende Stegbögen 7' und 7" auf, deren Scheitelpunkte jeweils in unterschiedliche Richtungen weisen. Wie Fig. 3 verdeutlicht, wechseln sich hierbei die Ausrichtungen der Scheitelpunkte der Elemente 22' bzw. ihrer Stegbögen 7', 7" (von links nach rechts in Fig. 3 gesehen) alternierend ab. Bezüglich aller übereinstimmender Merkmale kann ansonsten auf die Ausführungsform gemäß Fig. 2 verwiesen werden, insbesondere was den Aufbau der Stegmuster 5', 6' angeht. Fig. 3 zeigt den nicht-expandierten Zustand, während Fig. 4 den expandierten Zustand des Stegmusters 4' verdeutlicht, bei dem sich eine alternierend aufwärts- und abwärts ausgerichtete Anordnung der Kammern zwischen den Stegmustern und Elementen ergibt, wobei sich diese Definition aus einer Abfolge der Kammern gesehen von links nach rechts in Fig. 4 ergibt.

Sowohl die Ausführungsform gemäß Fig. 2 als auch gemäß der Fig. 3 und 4 kann durch das Schneiden der jeweiligen Stegmuster mittels einer Laseranordnung in ein Metallröhrchen hergestellt werden.

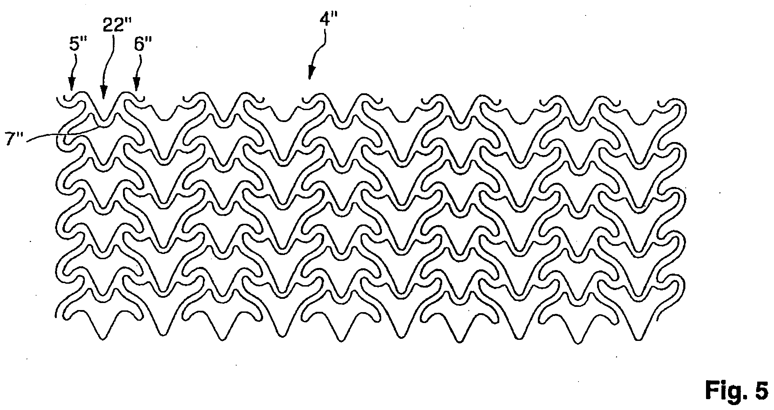

In Fig. 5 ist eine Ausführungsform einer Stegstruktur für den Stent gemäß Fig. 1 dargestellt, die im wesentlichen der Ausführungsform gemäß Fig. 2 entspricht. Daher sind die entsprechenden Bezugsziffern, die sich auf die Stegstruktur, die Stegmuster, die Stegbögen sowie die Federelemente beziehen, mit den gleichen Bezugsziffern wie in Fig. 2, jedoch doppelt gestrichen angegeben. Die Stegmuster 5", 6" sind stärker gewunden bzw. hinterschnitten als die Stegmuster 5, 6 der Fig. 2 und die Federelemente 22" spannen direkt mit den Stegmustern 5", 6" Zellen auf. In Ergänzung zu der schriftlichen Offenbarung wird explizit aus Offenbarungsgründen auf die Darstellung der Fig. 5 Bezug genommen.

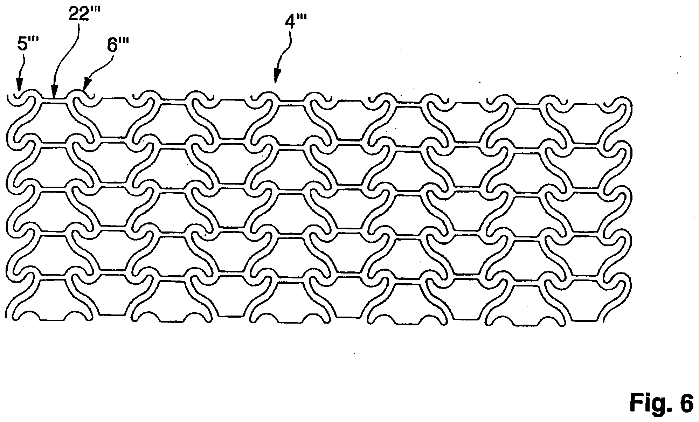

Fig. 6 stellt eine Variante der Ausführungsform gemäß Fig. 5 dar. Die Stegmuster 5"', 6"' entsprechen im wesentlichen der Ausführungsform gemäß Fig. 5. Die Federelemente 22"' sind jedoch als gerade Stege ausgebildet. Auch bei dieser Ausführungsform wird zur Offenbarung explizit auf die Darstellung der Fig. 6 Bezug genommen.

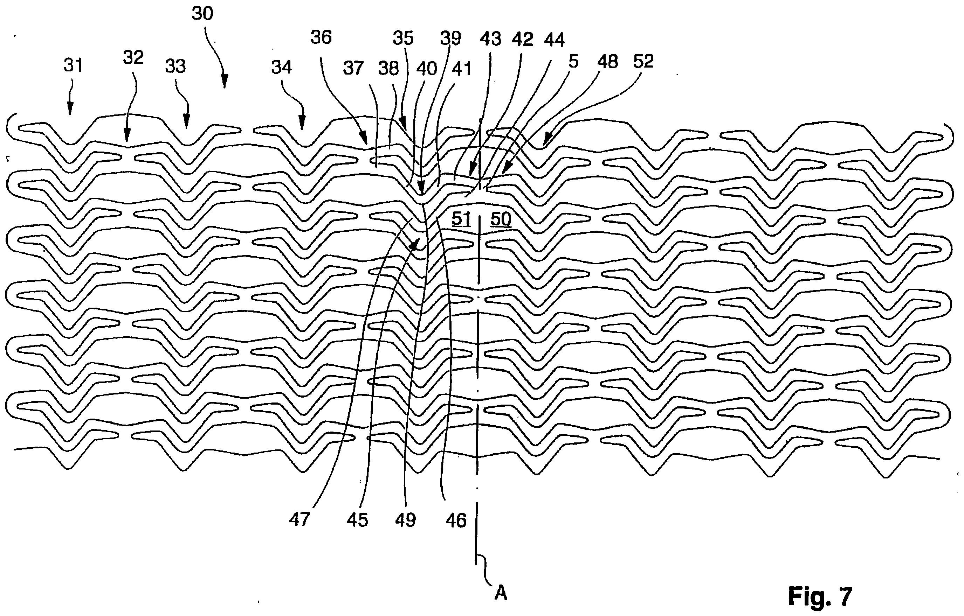

In Fig. 7 ist eine weitere Ausführungsform einer Stegstruktur 30 für einen Stent gemäß Fig. 1 dargestellt. Auch bei der Darstellung der Fig. 7 ist ein Ausschnitt der Wand des rohrförmigen Stegs in ebener Darstellung verdeutlicht. Der Stent mit dem Stegmuster gemäß Fig. 7 kann als ein Multizellularstent bezeichnet werden, der aus Rauten aufgebaut ist. Jede dieser Rauten weist in jedem Schenkel ein U- oder V-förmiges Federelement auf, welches im nicht gespreizten Zustand eine gewisse Flexibilität über der Längsachse garantiert.

Im einzelnen weist die Stegstruktur 30 eine Mehrzahl von nebeneinander angeordneten Stegmustern auf, von denen in Fig. 7 die Stegmuster 31, 33, 34, 35 und 52 mit entsprechenden Bezugsziffern gekennzeichnet sind. Diese Stegmuster bilden aufgrund der Rohrform des Stents radial verlaufende Wandstrukturen. Wie anhand des Beispieles der Stegmuster 31 und 33 in Fig. 7 verdeutlicht wurde, spannen diese mit einem Federelement 32 jeweils eine Zelle auf. Die Elemente 32 bilden hierbei, wie Fig. 7 verdeutlicht, integrale Bestandteile jeweils benachbarter Stegmuster. Anhand des Stegmusters 35 wird nachfolgend beispielhaft der Aufbau aller Stegmuster der Stegstruktur 30 erläutert.

Das Stegmuster 35 weist eine erste Schlaufe 36 auf, die zwei im spitzen Winkel zueinander angeordnete miteinander verbundene Schenkel 37, 38 aufweist. An die erste Schlaufe 36 schließt sich eine zweite Schlaufe 39 mit ebenfalls im spitzen Winkel zueinander angeordneten geraden Schenkeln 40, 41 an. An die Schlaufe 39 wiederum schließt sich eine dritte Schlaufe 42 an, die im spitzen Winkel zueinander angeordnete gerade Schenkel 43 und 44 aufweist. An die dritte Schlaufe 42 schließt sich eine vierte Schlaufe 45 mit wiederum geraden Schenkeln 46 und 47 an. Auch die Schenkel 46 und 47 der vierten Schlaufe 45 sind im spitzen Winkel zueinander angeordnet. Bei der in Fig. 7 dargestellten Ausführungsform bilden die Schenkel der jeweiligen Schlaufen 36, 39, 42 und 45 jeweils eine in etwa V-förmige Konfiguration. Grundsätzlich wäre auch eine U-förmige Konfiguration möglich. Wie Fig. 7 ferner verdeutlicht, wird ein komplettes Stegmuster natürlich durch eine immer wiederkehrende Abfolge von 4 Schlaufen in der zuvor beschriebenen Weise gebildet. Die Zahl der Schlaufen hängt von der jeweiligen Größe des Stents ab, so daß sich je nach Stentdurchmesser durch die Abfolge der Mehrzahl von vier Schlaufen geschlossene Ringe ergeben, die jeweils zusammen mit den Elementen 32 eine Zelle aufspannen.

Fig. 7 verdeutlicht ferner durch die Einzeichnung einer Achse A, daß das sich rechts an das zuvor beschriebene Stegmuster 35 anschließende Stegmuster 52 durch Spiegelung bzw. Umklappung des Stegmusters 35 um 180° ergibt. Somit ergibt sich für die gesamte Stegstruktur 30 stets eine Abfolge von jeweils an einer Achse A gespiegelten Stegmustern, wie dies zuvor am Beispiel der Stegmuster 35 und 52 beschrieben wurde.

Die Verbindung zwischen den Stegmustern 35 und 52 wird in Fig. 7 am Beispiel der Schlaufen 42 des Stegmusters 35 und 48 des Stegmusters 52 verdeutlicht. Es ergibt sich eine Verbindung im jeweiligen Scheitelpunkt S dieser Schlaufen 42 bzw. 48, wobei im Beispielsfalle die Scheitelpunkte S auf der Achse A liegen. Zu beiden Seiten der Achse A ergeben sich Zellen mit Teilräumen 50 und 51, die einen rautenähnlichen Gesamtraum bilden, der von den Schenkeln der zugeordneten Schlaufen begrenzt wird. Die Schlaufen 42 und 48 bilden somit ein weiteres Beispiel für ein Federelement entsprechend dem Element 32, das zuvor bereits erläutert wurde. In diesem Fall bilden die Elemente 32 aufgrund ihrer Ausbildung die den jeweiligen zellen zugeordneten Federelemente bzw. weisen derartige Federelemente auf:

Wie Fig. 7 schließlich verdeutlicht, weisen bei der dargestellten Ausführungsform alle Scheitelpunkte der sich gemäß der in Fig. 7 gewählten Darstellung nach oben öffnenden zweiten Schlaufen 39 in die gleiche Richtung, wobei zur Verdeutlichung der Scheitelpunkt 49 eingezeichnet ist. Im Beispielsfalle weisen aufgrund der in Fig. 7 gewählten Darstellung somit alle Scheitelpunkte nach unten.

Ferner weisen alle Scheitelpunkte der sich in Fig. 7 nach rechts öffnenden ersten Schlaufen 36 nach links, während die Scheitelpunkte der sich nach links öffnenden Schlaufen 42 nach rechts weisen. Jeweils umgekehrt ist es bei den gespiegelten Stegmustern, wie beispielsweise beim Stegmuster 52. Auch hier wird in Ergänzung zur schriftlichen Offenbarung zur Beschreibung und Offenbarung des Stegmusters 30 explizit auf die zeichnerische Darstellung der Fig. 7 verwiesen.

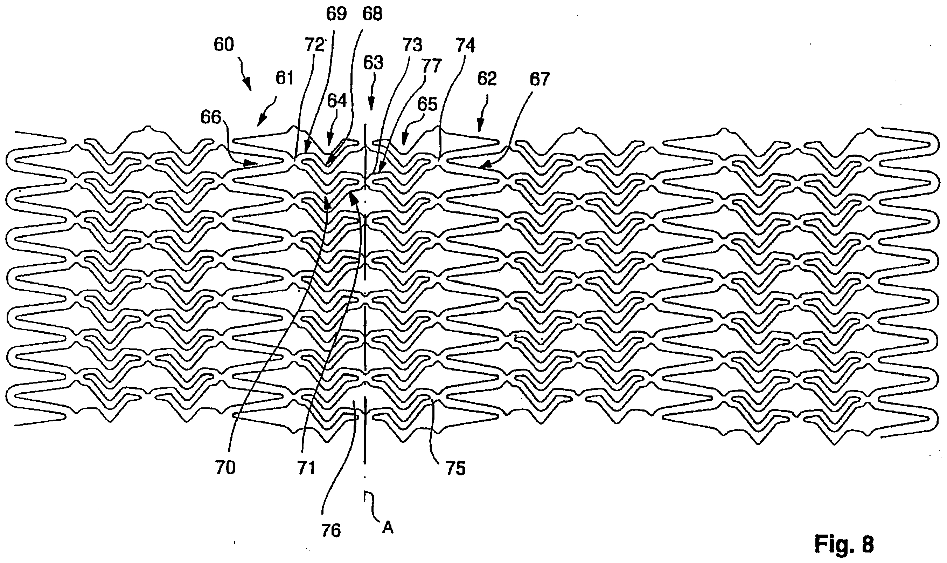

In Fig. 8 ist eine weitere Ausführungsform einer Stegstruktur 60 für den Stent gemäß Fig. 1 dargestellt. Auch diese Ausführungsform entspricht in ihrer Darstellung der Fig. 8 einer planen Darstellung eines Teils der Wand des Stents. Diese weitere Ausführungsform kann als ein Stent beschrieben werden, der einzelne radial verlaufende Stegmuster (mäanderartig bzw. entsprechend einer Sinusschwingung verlaufend) aufweist, die über Rauten miteinander gekoppelt sind, die ihrerseits in jedem Schenkel ein U- oder alternativ V-förmiges Federelement aufweisen, welches im nicht gespreizten Zustand eine erforderliche Flexibilität über der Längsachse ermöglicht. Vorteilhafterweise können die ersten Stegmuster (Mäander) in ihrer Stegbreite stärker ausgelegt werden als die mit ihnen (noch zu beschreibenden) eine Zelle aufspannenden Elemente, so daß durch die ersten Stegmuster eine hohe radiale Kraft aufgenommen werden kann. Im einzelnen ist die Stegstruktur 60 gemäß Fig. 8 wie folgt aufgebaut:

Es ist eine Mehrzahl von Stegmustern vorgesehen, von denen in der Fig. 8 beispielhaft die Stegmuster 61 und 62 mit Bezugszeichen gekennzeichnet sind. Wie Fig. 8 verdeutlicht, sind die Stegmuster 61, 62 nebeneinander angeordnet und weisen im wesentlichen parallele Achsen auf. Die Stegmuster 61 und 62 können exakt einer Sinusschwingung entsprechen oder zumindest einen ähnlichen Verlauf haben, so daß sie auch als mäandrierende bzw. gewunden verlaufende Stegmuster bezeichnet werden können. Sie sind jeweils 180° außer Phase angeordnet, was erläutert am Beispiele der Stegmuster 61 und 62 bedeutet, daß einer sich nach rechts öffnenden Schlaufe des Stegmusters 61 eine sich nach links öffnende Schlaufe des Stegmusters 62 gegenüberliegt. Es versteht sich, daß auch bei dieser Ausführungsform die Stegmuster 61, 62 im tatsächlichen röhrchenförmigen Zustand des Stents Ringe der Wand des Stents bilden, wobei natürlich entsprechend der Länge des Stents eine entsprechende Anzahl von Stegmustern 61, 62 vorgesehen ist. Nachfolgend wird jedoch zur Beschreibung stets auf die Stegmuster 61, 62 beispielhaft Bezug genommen.

Diese Stegmuster 61, 62 spannen zusammen mit Elementen (Federelementen) 63 Zellen auf.

Das Element 63 ist bei der dargestellten Ausführungsform aus zwei Federelement- bzw. Stegmustern 64, 65 aufgebaut. Wie Fig. 8 verdeutlicht, entstehen die Element-Stegmuster 64, 65 durch Spiegelung bzw. Klappung an einer Symmetrieachse A. Die Element-Stegmuster 64, 65 weisen jeweils vier aneinander sich anschliessende Schlaufen 68 bis 71 auf, deren Aufbau mit geradlinigen im spitzen Winkel zueinander angeordneten Schenkeln vergleichbar dem Aufbau in Fig. 7 ist, so daß diesbezüglich auf die vorangehende Beschreibung verwiesen werden kann. Die Schlaufen 68 bis 71 sind jeweils einstückig miteinander verbunden, so daß die Mehrzahl dieser Schlaufen die Struktur der Element-Stegmuster ergibt, zu deren Offenbarung wieder explizit auf Fig. 8 Bezug genommen wird.

Fig. 8 zeigt ferner, daß das Stegmuster 61 zusammen mit Knotenpunkten jeweils an den Scheitelpunkten seiner Schlaufen und mit dem Element 63 eine Zelle aufspannt. Repräsentativ ist ein Knotenpunkt 72 eingezeichnet. Die Element-Stegmuster 64 und 65 sind über eine Mehrzahl von Knotenpunkten miteinander verbunden, was durch den Knotenpunkt 73 symbolisiert ist. Das nachfolgende Stegmuster 62 spannt ebenfalls mit einer Mehrzahl von Knotenpunkten jeweils im Scheitelpunkt seiner Schlaufen und mit dem Element 63 eine Zelle auf, wobei wiederum repräsentativ ein Knotenpunkt 74 eingezeichnet ist. Wie Fig. 8 verdeutlicht, ist der Knotenpunkt 72 der Scheitelpunkt einer Schlaufe 66 des Stegmusters 61 während der Knotenpunkt 74 der Scheitelpunkt einer Schlaufe 67 des Stegmusters 62 ist.

Wie auch im Falle der Ausführungsform der Fig. 7 weisen die Scheitelpunkte der Schlaufen 66 bis 67 der Element-Stegmuster jeweils in die gleiche Richtung. Insofern kann auf die Ausführungen zu Fig. 7 Bezug genommen werden, wobei zur Vereinfachung des Verständnisses repräsentativ ein Scheitelpunkt 75 einer Schlaufe des rechten Element-Stegmusters 65 eingezeichnet ist.

Zu ergänzen ist ferner, daß die Schenkel der Schlaufen der Element-Stegmuster 64, 65 jeweils Zellen 76 mit zugeordneten Federelementen begrenzen. Auch hier ist in Fig. 8 repräsentativ eine Kammer 76 mit diesem Bezugszeichen versehen.

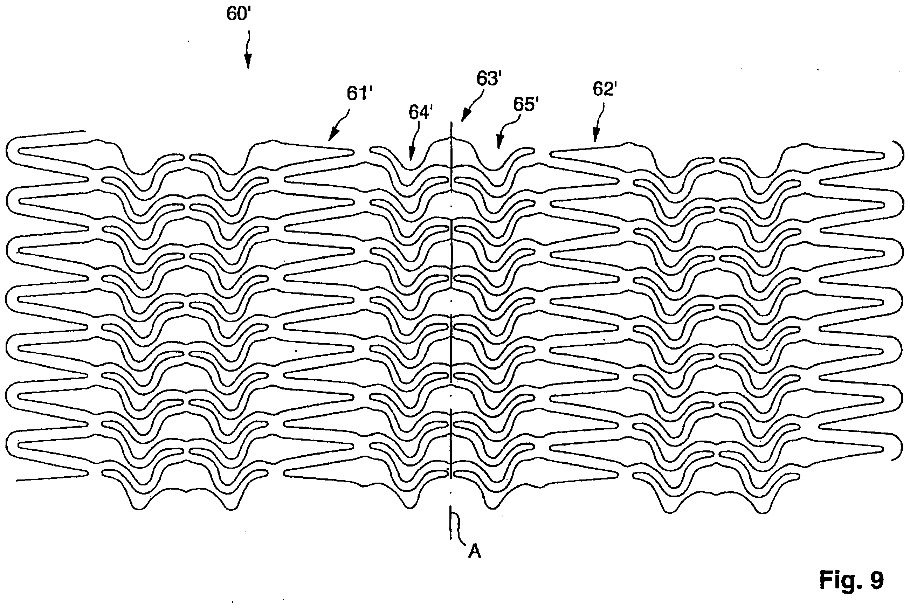

Fig. 9 entspricht im wesentlichen der Ausführungsform gemäß Fig. 8, so daß die Bezugsziffern der wesentlichen Elemente (einfach gestrichen) entsprechend der Ausführungsform gemäß Fig. 8 eingezeichnet sind. Die Fig. 9 verdeutlicht hierbei, daß das Element 63 bzw. dessen Schlaufen etwas mehr abgerundet als im Falle der Fig. 8 ausgebildet ist. Wie auch im Falle der vorhergehenden Ausführungsformen wird zur Offenbarung der Stegstruktur gemäß Fig. 9 explizit auf die Zeichnung Bezug genommen.

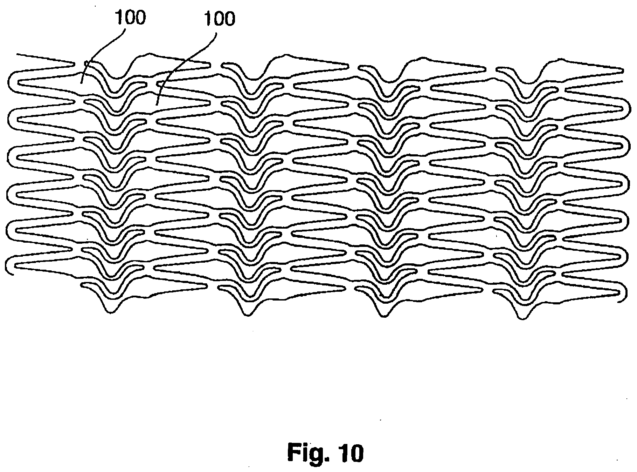

Schließlich zeigt Fig. 10 ein Fig. 9 ähnliches Ausführungsbeispiel eines erfindungsgemäßen Stents. Bei diesem Ausführungsbeispiel werden ausschließlich gleichförmige Zellen aufgespannt, wobei lediglich zwei Zellen repräsentativ mit dem Bezugszeichen 100 versehen sind.

Im Gegensatz zu den Ausführungsbeispielen 8 und 9, umschließen die Stege eine Zelle derart, daß jeweils ein Teil der Zelle aus geraden Stegen und ein zweiter Teil aus Stegen besteht, die jeweils ein Federelement aufweisen. Der linke Teil einer Zelle bildet jeweils den rechten Teil der links angrenzenden Zelle, der rechte Teil einer Zelle jeweils den linken Teil der rechts angrenzenden Zelle. Die rechts und links anschließenden Zellen sind jeweils um 180° gedreht, ansonsten aber deckungsgleich.

Fig. 10 beschreibt demnach einen Stent, welcher aus gleichförmigen Zellen aufgebaut ist, in der 2 D-Darstellung jeweils um 180° gedreht sind. Eine Zelle wird dabei immer von zwei geraden Schenkeln und zwei Schenkeln mit Federelementen aufgespannt. Die Federelemente in den Schenkeln ermöglichen eine hohe axiale Flexibilität im nicht expandierten Zustand.

Bezüglich der verschiedenen Komponenten, deren Ausgestaltung und Funktion dieses Ausführungsbeispiels wird auf obige Erläuterungen und Ausführungsformen verwiesen.

## Patentansprüche

1. Stent (1) mit einem rohrförmigen, flexiblen Körper (2), dessen Wand (3) eine Stegstruktur (4; 30; 60; 60') aufweist, wobei die Stegstruktur (4; 30; 60; 60') eine Mehrzahl von nebeneinander angeordneten Stegmustern (31, 33, 34, 35, 52) und eine Vielzahl von aneinander angrenzenden Zellen (18, 19; 50, 51; 76) aufweist, die von Stegen der Stegstruktur (4; 30; 60; 60') umgrenzt sind, wobei pro Zelle (18, 19; 50, 51; 76) zumindest ein Federelement (22; 32; 63; 63') vorgesehen ist, und wobei die Stegmuster folgendes aufweisen:
erste Schlaufen (36), die sich in eine erste Richtung öffnen,
zweite Schlaufen (39), die sich an die ersten Schlaufen (36) anschließen und die sich in eine zweite Richtung öffnen,
dritte Schlaufen (42), die sich an die zweiten Schlaufen (39) anschließen und sich in eine dritte, zur ersten Richtung entgegengesetzte Richtung öffnen, und
vierte Schlaufen (45), die sich an die dritten Schlaufen (42) anschließen und sich in dieselbe Richtung öffnen wie die zweiten Schlaufen (39),
wobei die ersten bis vierten Schlaufen (36, 39, 42, 45) jeweils zwei im spitzen Winkel zueinander angeordnete, miteinander verbundene Schenkel (37; 38; 40; 41; 43; 44; 46; 47) aufweisen und eine wiederkehrende Abfolge von Schlaufen bilden, bei der sich die ersten Schlaufen einer Abfolge an die vierten Schlaufen einer vorhergehenden Abfolge anschließen,
**dadurch gekennzeichnet, dass** die Stegstruktur (4; 30; 60; 60') aus einer Abfolge von jeweils an einer Achse (A) gespiegelten Stegmustern (31, 33, 34, 35, 52) gebildet wird, deren Verbindung jeweils an den Scheitelpunkten der ersten bzw. dritten Schlaufen besteht.

2. Stent nach Anspruch 1, **dadurch gekennzeichnet, dass** eine oder mehrere Schlaufen (36, 39, 42, 45) V-förmig ausgebildet sind.

3. Stent nach Anspruch 1, **dadurch gekennzeichnet, dass** eine oder mehrere Schlaufen (36, 39, 42, 45) U-förmig ausgebildet sind.

4. Stent nach einem der Ansprüche 1 bis 3, **dadurch gekennzeichnet, dass** ein Federelement (32) durch zwei mit ihren Scheitelpunkten (S) aneinander angrenzende Schlaufen (42, 48) gebildet wird.

5. Stent nach Anspruch 4, **dadurch gekennzeichnet, dass** die Zellen zu beiden Seiten der Achse (A) angeordnete Teilräume (50, 51) aufweisen.

6. Stent nach Anspruch 5, **dadurch gekennzeichnet, dass** die Teilräume (50, 51) einen rautenähnlichen oder flügelähnlichen Gesamtraum bilden.

7. Stent nach einem der Ansprüche 1 bis 6, **dadurch gekennzeichnet, dass** die Scheitelpunkte (49) der zweiten Schlaufen (39) und der vierten Schlaufen (45) alle in die gleiche Richtung weisen.

8. Stent nach einem der Ansprüche 1 bis 7, **dadurch gekennzeichnet, dass** alle Scheitelpunkte der ersten Schlaufen (36) und alle Scheitelpunkte der dritten Schlaufen (42) in entgegengesetzte Richtungen weisen.

## Claims

1. Stent (1) comprising a tubular, flexible body (2) whose wall (3) has a web structure (4; 30; 60; 60'), wherein the web structure (4; 30; 60; 60') has a plurality of adjacent web patterns (31, 33, 34, 35, 52) and a plurality of adjoining cells (18, 19; 50, 51; 76) that are surrounded by webs of said web structure (4, 30; 60; 60'), wherein at least one spring element (22; 32; 63; 63') is provided per cell (18, 19; 50, 51; 76), and wherein the web pattern comprises the following:
first loops (36) that open in a first direction,
second loops (39) that follow the first loops (36) and open in a second direction,
third loops (42) that follow the second loops (39) and open in a third direction that is opposite to the first direction, and
fourth loops (45) that follow the third loops (42) and open in the same direction as the second loops (39),
wherein the first to fourth loops (36, 39, 42, 45) comprise in each case two interconnected legs (37; 38; 40; 41; 43; 44, 46, 47) that are arranged at an acute angle with respect to one another and form a recurring sequence of loops in which the first loops of a sequence follow the fourth loops of a preceding sequence,
**characterized in that** the web structure (4; 30; 60 60') is formed from a sequence of in each case web patterns (31, 33, 34, 35, 52) that are mirror-inverted at an axis (A), the connection therebetween being provided in each case at the vertices of the first or third loops.

2. Stent according to claim 1, **characterized in that** one or multiple loops (36, 39, 42, 45) are formed in a V-shaped manner.

3. Stent according to claim 1, **characterized in that** one or multiple loops (36, 39, 42, 45) are formed in a U-shaped manner.

4. Stent according to any one of claims 1 to 3, **characterized in that** a spring element (32) is formed by means of two loops (42, 48) that adjoin one another with their vertices (S).

5. Stent according to claim 4, **characterized in that** the cells comprise partial chambers (50, 51) that are arranged on both sides of the axis (A).

6. Stent according to claim 5, **characterized in that** the partial chambers (50, 51) form a diamond-like or wing-like entire chamber.

7. Stent according to any one of claims 1 to 6, **characterized in that** the vertices (49) of the second loops (39) and of the fourth loops (45) all face the identical direction.

8. Stent according to any one of claims 1 to 7, **characterized in that** all vertices of the first loops (36) and all vertices of the third loops (42) face opposite directions.

## Revendications

1. Stent (1) avec un corps (2) flexible tubulaire, dont la paroi (3) présente une structure de nervure (4 ; 30 ; 60 ; 60'), dans lequel la structure de nervure (4 ; 30 ; 60 ; 60') présente une pluralité de modèles de nervure (31, 33, 34, 35, 52) agencés les uns à côté des autres et une pluralité de cellules (18, 19 ; 50, 51 ; 76) contiguës les unes à côté des autres qui sont délimitées par des nervures de la structure de nervure (4 ; 30 ; 60 ; 60'), dans lequel au moins un élément de ressort (22 ; 32 ; 63 ; 63') est prévu par cellule (18, 19 ; 50, 51 ; 76) et dans lequel les modèles de nervure présentent:
des premières boucles (36) qui s'ouvrent dans un premier sens,
des deuxièmes boucles (39) qui se raccordent aux premières boucles (36) et qui s'ouvrent dans un deuxième sens,
des troisièmes boucles (42) qui se raccordent aux deuxièmes boucles (39) et s'ouvrent dans un troisième sens opposé au premier sens, et
des quatrièmes boucles (45) qui se raccordent aux troisièmes boucles (42) et s'ouvrent dans le même sens que les deuxièmes boucles (39),
dans lequel les premières à quatrièmes boucles (36, 39, 42, 45) présentent respectivement deux branches (37 ; 38 ; 40 ; 41 ; 43 ; 44 ; 46 ; 47) reliées entre elles, agencées en angle aigu l'une par rapport à l'autre et forment une suite récurrente de boucles, pour laquelle les premières boucles d'une suite se raccordent aux quatrièmes boucles d'une suite précédente,
**caractérisé en ce que** la structure de nervure (4 ; 30 ; 60 ; 60') est formée d'une suite de modèles de nervure (31, 33, 34, 35, 52) respectivement reflétés sur un axe (A), dont la liaison existe respectivement sur les sommets des premières ou troisièmes boucles.

2. Stent selon la revendication 1, **caractérisé en ce qu'**une ou plusieurs boucles (36, 39, 42, 45) sont réalisées en V.

3. Stent selon la revendication 1, **caractérisé en ce qu'**une ou plusieurs boucles (36, 39, 42, 45) sont réalisées en U.

4. Stent selon l'une des revendications 1 à 3, **caractérisé en ce qu'**un élément de ressort (32) est formé par deux boucles (42, 48) contiguës l'une à l'autre avec leurs sommets (S).

5. Stent selon la revendication 4, **caractérisé en ce que** les cellules présentent des espaces partiels (50, 51) agencés des deux côtés de l'axe (A).

6. Stent selon la revendication 5, **caractérisé en ce que** les espaces partiels (50, 51) forment un espace entier similaire à un losange ou une aile.

7. Stent selon l'une des revendications 1 à 6, **caractérisé en ce que** les sommets (49) des deuxièmes boucles (39) et des quatrièmes boucles (45) sont tous tournés dans le même sens.

8. Stent selon l'une des revendications 1 à 7, **caractérisé en ce que** tous les sommets des premières boucles (36) et tous les sommets des troisièmes boucles (42) sont tournés dans des sens opposés.
